# EUROPEAN PATENT APPLICATION

(11) **EP 4 707 380 A1**
(43) Date of publication of application: **11.03.2026**
(21) Application number: 24811226.0
(22) Date of filing: 21.02.2024
(51) Int. Cl.: C12N 1/20, C12P 19/56, C12R 1/225

(54) **LACTOBACILLUS MALI STRAIN AND USES THEREOF**

(30) Priority: 23.05.2023 KR 20230066300
(71) Applicant: CJ Cheiljedang Corporation, Seoul 04560 (KR)
(72) Inventor: JEONG, Goo-Yeong, Seoul 04560 (KR); PARK, Ye-Lim, Seoul 04560 (KR); PARK, Sunghee, Seoul 04560 (KR); KIM, Jungeun, Seoul 04560 (KR); SHIM, Dongseok, Seoul 04560 (KR)
(74) Representative: Meissner Bolte Partnerschaft mbB
(86) International application number: PCT/KR2024/002237
(87) International publication number: WO 2024/242287

(57) **Abstract**

The present invention relates to a novel *Lactobacillus mali* CJST01 strain that has excellent steviol glycoside transglycosylation activity, and a method for producing steviol glycoside using same. The novel *Lactobacillus mali* CJST01 strain has excellent steviol glycoside transglycosylation activity compared to *Lactobacillus mali* DSM20444 strain which is known to produce steviol glycoside transglycosylation, thereby shortening an enzyme reaction time in a manufacturing process. The steviol glycoside produced by using the strain has an advantage of having better taste quality or flavor than alpha-1,4-linked steviol glycoside and rebaudioside M. In addition, the transglycosylated rebaudioside A produced using the *Lactobacillus mali* CJST01 strain has reduced off-taste and off-flavor and excellent solubility.

## Description

### [TECHNICAL FIELD]

### Cross-reference to related application(s)

The present application claims the benefit of the priority based on Korean Patent Application No. 10-2023-0066300 filed on May 23, 2023, and the entire contents disclosed in the documents of the corresponding Korean patent application are incorporated as a part of the present description.

The present invention relates to a novel *Lactobacillus mali* strain with excellent transglycosylated steviol glycoside production ability and a method for preparing transglycosylated steviol glycosides using the same.

### [BACKGROUND ART]

As the World Health Organization (WHO) recommends lowering daily sugar intake due to concerns about diseases (obesity, etc.) according to sugar intake, centering developed countries, various policies to reduce sugar intake are being actively discussed under the leadership of governments. Accordingly, needs for various alternative sweetener materials are increasing in the market, and alternative sweetener materials are being continuously developed and commercialized. Currently, as an alternative sweetener for replacing sugar, a synthetic high intensity sweetener (Saccharin, Aspartame, Sucralose, etc.) are mainly used, but consumer demand for development of natural alternative sweeteners has been growing, as concerns about safety of synthetic sweeteners continue to rise.

Stevia, a natural high sweetener that has recently attracted attention, refers to a sweetener derived from leaves of the plant called *Stevia rebaudiana* Bertoni. Steviol glycoside, known as a main sweetening ingredient, has sweetness of 200∼400 times of sugar(sucrose) for each ingredient, and rebaudioside A, rebaudioside D, and rebaudioside M are mainly used as food and beverage sweeteners. However, rebaudioside A has unique bitterness, and rebaudioside D and rebaudioside M have low solubility and expensive price, so they have a disadvantage of limits in application, and therefore, they have limitations in use.

In order to solve this problem, transglycosylated rebaudioside A produced using *Lactobacillus mali* has been studied, and this is a structure in which 1~4 glucoses are combined with alpha-1,6 to rebaudioside A, and it has been confirmed that sensory characteristics are improved, by confirming the improvement in bitterness and excellence in sweetness preference compared to rebaudioside A, a steviol glycoside.

*Lactobacillus mali* is a microorganism found in fermented foods such as apple wine, wine and the like. The present inventors have confirmed that the newly discovered *Lactobacillus mali* CJST01 strain from various fermented foods is significantly excellent compared to the *Lactobacillus mali* DSM20444 strain known to produce a transglycosylated steviol glycoside, thereby completing the present application.

### [DISCLOSURE]

### [TECHNICAL PROBLEM]

One embodiment of the present invention provides a *Lactobacillus mali* CJST01 strain.

Another embodiment of the present invention provides a method for preparing transglycosylated steviol glycosides using the strain.

Other embodiment of the present invention provides a transglycosylated steviol glycoside prepared by the method for preparing.

Other embodiment provides a sweetener comprising the prepared transglycosylated steviol glycosides.

Other embodiment provides a composition for producing transglycosylated steviol glycosides, comprising the strain or a culture thereof.

Other embodiment provides a use of a *Lactobacillus mali* CJST01 strain or a culture thereof, for preparing a transglycosylated steviol glycosides and/or a sweetener comprising transglycosylated steviol glycosides.

Other embodiment provides a use of the composition comprising a *Lactobacillus mali* CJST01 strain or a culture thereof, for preparing a transglycosylated steviol glycosides and/or a sweetener comprising transglycosylated steviol glycosides.

### [TECHNICAL SOLUTION]

This is explained in detail as follows. On the other hand, each description and embodiment disclosed in the present invention can be applied to each other description and embodiment. In other words, all combinations of various elements disclosed in the present invention fall within the scope of the present invention. In addition, the scope of the present application cannot be considered limited by the specific description described below.

The present application provides a novel *Lactobacillus mali* strain. The novel *Lactobacillus mali* strain may be the strain having transglycosylated steviol glycoside production ability

In the present application, as one embodiment, as a result of confirming stevioside transglycosylation ability by obtaining crude enzyme solution from culture solution cultured by homogenizing fermented foods to discover a strain with excellent transglycosylated steviol glycoside production ability, a *Lactobacillus mali* strain with excellent stevioside transglycosylation activity was selected, and this was named *Lactobacillus mali* CJST01 strain, and deposited to Korea Research Institute of Bioscience and Biotechnology, Korean Collection for Type Cultures (KCTC), which is an international depository authority under Budapest Treaty on May 8th, 2023, and given accession number KCTC15433BP.

Therefore, the novel *Lactobacillus mali* strain may be a *Lactobacillus mali* CJST01 strain deposited with accession number KCTC15433BP.

The *Lactobacillus mali* CJST01 strain has 16S rRNA of SEQ ID NO: 1.

The term in the present application, "steviol glycoside" is one of natural sweeteners, and has a form in which glucose, rhamnose, xylose and the like are combined to the 13th, 19th-OH of Chemical formula 1 (steviol).

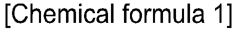

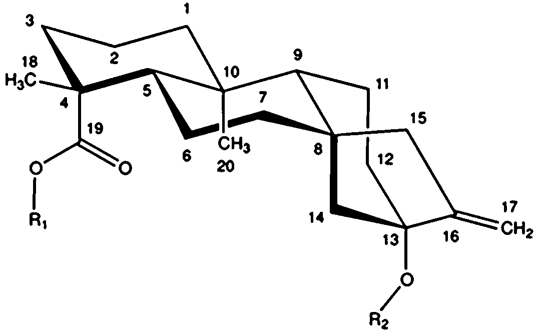

In general, in Chemical formula 1, to R1, hydrogen (H) may be combined, or 1 to 3 glucoses may be combined by a β bond, and to R2, 1 glucose, or xylose, or rhamnose may be combined by a β bond, and thereto, 0 to 2 glucoses may be combined by a β bond, but not limited thereto.

α-/β- glycoside bonds are distinguished by relative stereochemistry (R-type or S-type) of an anomeric position and a stereocenter furthest from carbon 1 of a monosaccharide. In general, when two carbons have the same stereochemistry, an α-glycoside bond is formed, whereas a β-glycoside bond occurs when two carbons have different stereochemistry.

In the present application, the term "transglycosylated steviol glycoside" may be in a form in which 1 to 9 glucoses are added by α-bonds at the 19-OH position of a steviol glycoside by a *Lactobacillus mali* strain, using sucrose and a steviol glycoside as a substrate, and more specifically, may be in a form in which 1 to 9 glucoses are added by α-(1, 6) bonds to a glucose combined to the 19-OH position of a steviol glycoside, but not limited thereto.

In the present application, the steviol glycoside may be any one selected from the group consisting of stevioside, rebaudioside A, rebaudioside C, rebaudioside F, dulcoside A, steviolbioside, and rubusoside, but not limited thereto.

The *Lactobacillus mali* CJST01 strain may have excellent transglycosylated steviol glycoside production ability compared to a known *Lactobacillus mali* strain.

In the present application, the term "transglycosylated steviol glycoside production ability" means ability capable of producing transglycosylated steviol glycoside by transferring glucose to steviol glycoside in a glucose donor, and may be interchangeably used with "transglycosylated steviol glycoside productivity" or "transglycosylated steviol glycoside conversion rate", and the transglycosylated steviol glycoside production ability, productivity, or conversion rate may be confirmed by reacting crude enzyme solution obtained after culturing a strain with a steviol glycoside and sucrose by a reducing sugar determination method (DNS method), HPLC analysis method, or LC-MS analysis method, but not limited thereto.

In one embodiment of the present application, it was confirmed that the *Lactobacillus mali* CJST01 strain had a higher conversion rate from a steviol glycoside to a transglycosylated steviol glycoside than other microorganisms comprising a known enzyme under the same condition.

Furthermore, the present application provides a method for preparing transglycosylated steviol glycosides using a *Lactobacillus mali* CJST01 strain.

The *Lactobacillus mali* CJST01 strain, and the transglycosylated steviol glycoside are same as described above.

The method for preparing transglycosylated steviol glycosides may comprise reacting sucrose and a steviol glycoside, in the presence of a *Lactobacillus mali* strain or a culture thereof.

In the present application, the culture refers to a culture solution comprising microbial cells or crude enzyme solution excluding microbial cells, and the "culture" includes various substances discharged into a medium during growth by a microorganism with medium components composed for culture of microorganisms, and specifically, transglycosylase having sucrose hydrolytic activity is comprised. The enzyme having sucrose hydrolytic activity may play a role of decomposing sucrose into glucose or α binding 1 to 9 glucoses via α-(1, 6) bonds at the 19-OH position of a steviol glycoside, but not limited thereto.

The medium used for culture should meet requirements of a specific strain. Culture media for *Lactobacillus mali* strains are known. For example, the microorganism of the present application may be cultured while adjusting temperature, pH and the like under an aerobic condition in a common medium containing a carbon source, a nitrogen source, an amino acid, a vitamin, and the like, which are appropriate. At this time, as the carbon source, carbohydrates such as glucose, fructose, and sucrose, amino acids such as glutamic acid, and cysteine, and the like are comprised. Specifically, natural organic nutrients such as starch hydrolysate, and molasses may be used, and preferably, it is a carbohydrate such as glucose, fructose, sterilized pre-treated molasses (namely, molasses converted into reducing sugar), and other appropriate amounts of carbon sources may be used variously without limitation, but not limited thereto. As the nitrogen source, inorganic nitrogen sources such as ammonia; and organic nitrogen sources such as amino acids including glutamic acid and cysteine and peptone, meat extract, yeast extract and the like may be used. These nitrogen sources may be used alone or in combination, but not limited thereto. For the medium, as the phosphorus source, phosphoric acid, potassium dihydrogen phosphate or dipotassium hydrogen phosphate or corresponding sodium-containing salts may be used, but not limited thereto. As an inorganic compound, magnesium sulfate, iron sulfate, manganese sulfate and calcium chloride and the like may be used, and in addition, amino acids, vitamins and appropriate precursors and the like may be included. These media or precursors may be added to culture in a batch or continuous manner, but not limited thereto.

During culture, by adding a compound such as potassium hydroxide, ammonia and phosphoric acid to a culture in an appropriate method, the pH of the culture may be adjusted. In addition, during culture, foarming generation may be inhibited using an antifoaming agent such as fatty acid polyglycol ester. Moreover, in order to maintain an aerobic condition of the culture, oxygen or oxygen-containing gas may be injected into the culture. The temperature of the culture may be 27°C to 37°C, specifically, 30°C to 33°C. The culture period may continue until the desired yield of a useful material is obtained, and specifically, it is 20 to 120 hours.

The method for preparing transglycosylated steviol glycosides may include preparing each of a composition including a *Lactobacillus mali* strain or a culture thereof, sucrose, and steviol glycoside; and mixing the composition.

In one embodiment of the present application, the preparation method may include adding an additive to at least one of the compositions above.

In another embodiment, the additive may be a cryoprotectant or excipient, and the method may further include a freeze-drying step after adding the additive. The cryoprotectant or excipient are as described below.

The steviol glycoside may be any one selected from the group consisting of stevioside, rebaudioside A, rebaudioside C, rebaudioside F, dulcoside A, steviolbioside, and rubusoside, but not limited thereto.

In addition, the present application provides a transglycosylated steviol glycoside prepared by the method for preparing transglycosylated steviol glycosides, and a sweetener comprising the transglycosylated steviol glycosides.

The prepared transglycosylated steviol glycosides and the sweetener comprising the same may have reduced off-taste and off-flavor, and improved sweetness quality.

In addition, the present application provides a composition for producing transglycosylated steviol glycosides comprising a *Lactobacillus mali* CJST01 strain or a culture thereof.

The *Lactobacillus mali* CJST01 strain, and the transglycosylated steviol glycoside are same as described above.

The composition may further include a cryoprotectant or an excipient. The cryoprotectant or excipient may be a non-naturally occurring substance or a naturally occurring substance, but is not limited thereto. In another embodiment, the cryoprotectant or excipient may be a substance that does not naturally contact with the *Lactobacillus mali* CJST01, or a substance that is not naturally contained simultaneously with the strain, but is not limited thereto. In still another embodiment, the composition may further include at least one cryoprotectant selected from the group consisting of glycerol, trehalose, maltodextrin, skim milk powder and starch, and/or at least one excipient selected from the group consisting of glucose, dextrin and skim milk. The cryoprotectant of the present disclosure may be contained in an amount of 0.01% to 20% by weight and 0.01% to 10% by weight based on the total weight of the composition. Specifically, the glycerol may be contained in an amount of 5% to 20% by weight, the trehalose may be contained in an amount of 2% to 10% by weight, the maltodextrin may be contained in an amount of 2% to 10% by weight, the skim milk powder may be contained in an amount of 0.5% to 2% by weight, and the starch may be contained in an amount of 0.1% to 1% by weight in the composition. In addition, the excipient may be contained in an amount of 75% to 95% by weight or 85% to 95% by weight based on the total weight of the composition.

The composition may be used in forms of liquid, granule, or powder, but is not limited thereto.

The present application provides a use of a *Lactobacillus mali* CJST01 strain or a culture thereof, for preparing a transglycosylated steviol glycosides and/or a sweetener comprising transglycosylated steviol glycosides.

The present application provides a use of the composition comprising a *Lactobacillus mali* CJST01 strain or a culture thereof, for preparing a transglycosylated steviol glycosides and/or a sweetener comprising transglycosylated steviol glycosides.

The *Lactobacillus mali* CJST01 strain, and the transglycosylated steviol glycoside are same as described above.

### [ADVANTAGEOUS EFFECTS]

The present invention relates to a novel *Lactobacillus mali* strain and a method for preparing transglycosylated steviol glycosides using the same, and the novel *Lactobacillus mali* CJST01 strain can shorten enzyme reaction time during the manufacturing process, due to more excellent steviol glycoside transglycosylation activity than *Lactobacillus mali* DSM20444 strain which is known to produce transglycosylated steviol glycosides, and high productivity, and the steviol glycoside produced using the strain has an advantage that the taste quality or flavor is excellent compared to steviol glycoside and rebaudioside M combined with alpha-1,4. In addition, transglycosylated rebaudioside A produced using the *Lactobacillus mali* CJST01 strain has reduced off-taste and off-flavor, and excellent solubility.

### [BRIEF DESCRIPTION OF THE DRAWINGS]

FIG. 1 is a drawing which shows the result of comparing the result of measuring sucrose hydrolyzing activity of the *Lactobacillus mali* CJST01 strain with the control group, the *Lactobacillus mali* DSM20444 strain, using DNS method.
FIG. 2 is a drawing which shows the result of comparing the conversion rate of transglycosylated rebaudioside A from rebaudioside A by the amount of crude enzyme solution added of the *Lactobacillus mali* CJST01 strain and the control group, the *Lactobacillus mali* DSM20444 strain.
FIG. 3 is a drawing which shows the chromatogram, which the reaction solution after adding the crude enzyme solution of 15% of the *Lactobacillus mali* CJST01 strain and the control group, the *Lactobacillus mali* DSM20444 strain, is analyzed by HPLC.
FIG. 4 is a drawing which shows the result of HPLC analysis of the reaction solution components reacted after adding the *Lactobacillus mali* CJST01 crude enzyme solution at a concentration of 10% by concentrating it 5 times to rebaudioside A 10% and sucrose 10%.
FIG. 5 is a drawing which shows the result of analyzing the reaction solution components reacted after adding the *Lactobacillus mali* CJST01 crude enzyme solution at a concentration of 10% by concentrating it 5 times to rebaudioside A 10% and sucrose 10%, with LC-MS.
FIG. 6 is a drawing which shows the result of analyzing the reaction solution components reacted after adding the *Lactobacillus mali* CJST01 crude enzyme solution at a concentration of 10% by concentrating it 5 times to rebaudioside A 10% and sucrose 10%, with LC-MS.
FIG. 7 is a drawing which shows the chromatogram of comparing the before and after the reaction by steviol glycoside of the *Lactobacillus mali* CJST01 crude enzyme solution.
FIG. 8 is the intensity analysis result for detailed flavor properties and the like of transglycosylated rebaudioside A prepared with the *Lactobacillus mali* CJST01 crude enzyme solution.

### [MODE FOR INVENTION]

Hereinafter, the present invention will be described in more detail by the following examples. However, these examples are intended to illustrate the present invention only, but the scope of the present invention is not limited by these examples.

### Example 1. Selection of a novel strain having transglycosylation activity on stevioside

The microbial library stock possessed by our company, which was isolated from samples collected from traditional fermented foods and manufacturing processes such as kimchi, fermented soybean pastes and sauces, vinegar, fermented liquor and the like, was smeared on MRS agar media and cultured at 30°C for 24 hours, and among them, strains with a rapid growth rate were selected by naked eyes and colonies were inoculated to MRS broth 3mL and then cultured at 30°C for 24 hours. Each culture solution was centrifuged at 8000 rpm for 10 minutes and microbial cells and supernatant were isolated and then the supernatant (crude enzyme solution) was recovered. For each crude enzyme solution, sucrose hydrolyzing ability was confirmed through DNS method, and the stevioside transglycosylation ability was confirmed by reacting sucrose and stevioside by 5% each, and shown in Table 1.

**[Table 1]**

| Strain name | Sucrose hydrolyzing ability | Stevioside Transglycosylation ability |
|---|---|---|
| *Bifidobacterium bifidum* | × | × |
| *Lactobacillus reuteri* | ○ | × |
| *Lactobacillus casei* | ○ | × |
| *Lactobacillus plantarum* | ○ | × |
| *Lactobacillus salivarius* | ○ | × |
| ***Lactobacillus mali*** | ○ | ○ |

As shown in Table 1, a total of 6 kinds of strains, which are Bifidobacterium bifidum, Lactobacillus reuteri, Lactobacillus casei, Lactobacillus plantarum, Lactobacillus salivarius and *Lactobacillus mali,* were selected. As a result of confirming the sucrose hydrolytic activity and stevioside transglycosylation activity, it was confirmed that among the 6 kinds of strains, 5 kinds had the sucrose hydrolytic activity, but among them, only the *Lactobacillus mali* strain had the stevioside transglycosylation activity.

### Example 2. Identification of 16S rRNA of selected Lactobacillus mali strain

As a result of confirming the 16S rRNA sequence of the *Lactobacillus mali* strain selected in Example 1, it was confirmed that there was no strain having the same sequence, and it was different from the base sequences of the known *Lactobacillus mali* strains. Accordingly, it was confirmed that the *Lactobacillus mali* strain selected in Example 1 is a novel *Lactobacillus mali* strain, and the strain name was named *Lactobacillus mali* CJST01, and the 16S rRNA sequence was registered in NCBI GenBank.

In addition, the *Lactobacillus mali* CJST01 strain was deposited to Korea Research Institute of Bioscience and Biotechnology, Korean Collection for Type Cultures (KCTC), which is an international depository authority under Budapest Treaty on May 8th, 2023, and given accession number KCTC15433BP.

### Example 3. Confirmation of sucrose hydrolytic activity of Lactobacillus mali CJST01 strain

In order to confirm that the novel *Lactobacillus mali* CJST01 strain has excellent sucrose hydrolytic activity, it was compared to the sucrose hydrolytic activity of *Lactobacillus mali* DSM20444 strain known to have sucrose hydrolytic activity.

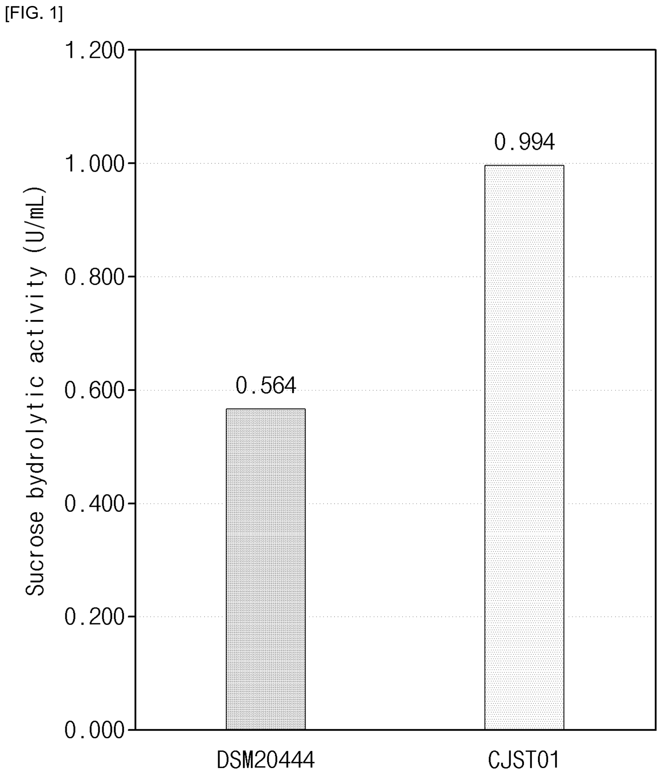

Specifically, after culturing 2 kinds of the *Lactobacillus mali* CJST01 strain and *Lactobacillus mali* DSM20444 strain in MRS broth in which sucrose was added, respectively, the culture solution was centrifuged at 8000 rpm for 10 minutes and microbial cells and supernatant were isolated and then only the supernatant (crude enzyme solution) was taken. The obtained crude enzyme solution was reacted with sucrose, and the sucrose hydrolytic activity was measured through reducing sucrose determination (DNA method) and shown in FIG. 1. After 200 mM of sucrose aqueous solution and each of the fractions were mixed to 50 mM sodium acetate buffer at 1:1, they were reacted in 40°C water bath for 10 minutes and then deactivated at 100°C. DNS reagents were added at a ratio of 1:3 and mixed and reacted at 100°C for 5 minutes, and then DNS reaction was stopped on ice immediately. After that, the absorbance (OD) of the DNA reagents was measured at 575 nm, and then the activity was confirmed by substituting into the prepared fructose standard curve equation. Then, 1 Unit is defined as the amount (mL) of enzyme that increases 1µmole of reducing sucrose per 1 minute.

As a result, as shown in FIG. 1, it was confirmed that the novel *Lactobacillus mali* CJST01 strain showed sucrose hydrolytic activity about 1.7 times higher compared to the *Lactobacillus mali* DSM20444 strain.

### Example 4. Evaluation of conversion from rebaudioside A into transglycosylated rebaudioside A

The conversion rate from rebaudioside A into transglycosylated rebaudioside A of the *Lactobacillus mali* CJST01 strain and *Lactobacillus mali* DSM20444 strain crude enzyme solution prepared in Example 3 was compared as follows.

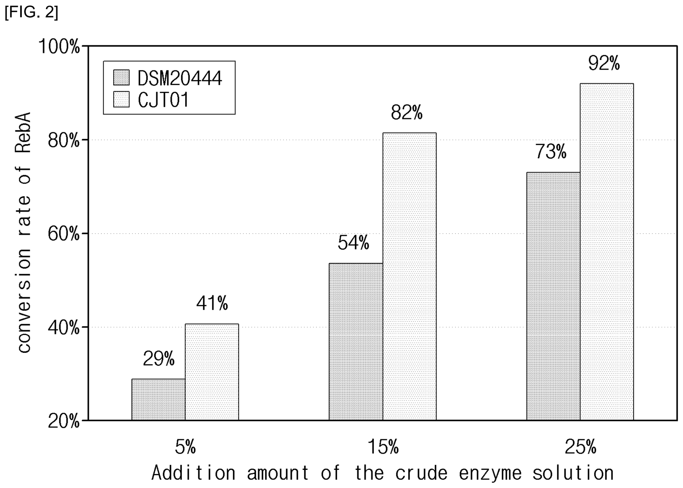

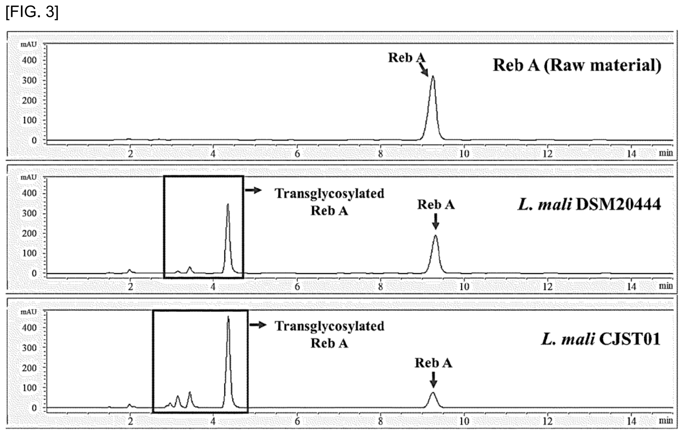

Specifically, rebaudioside A (Daepyung) and sucrose (CJ CheilJedang) were added to 50 µM sodium acetate buffer (pH 5.0) by 10% each, and the crude enzyme solution of 2 kinds of the *Lactobacillus mali* strains prepared in Example 3 was added by 5%, 15%, or 25%, respectively, and reacted at 40°C for 24 hours. After reacting, they were deactivated at 100°C, and the result of confirming the conversion rate into transglycosylated rebaudioside A by HPLC was shown in FIG. 2. In addition, after the addition reaction of 15% of the crude enzyme solution, each reaction solution was compared by a chromatogram, and is shown in FIG. 3. HPLC analysis conditions were shown in Table 2 below.

**[Table 2]**

| | |
|---|---|
| Detector | UV 210 nm |
| Column | Capcell pak C18 MG II (4.6 x 250, 5 um) |
| Column temp. | 40°C |
| Mobile phase | 30% acetonitrile |
| | isocratic |
| Flow rate | 1.0 mL/min |

As a result of comparing the conversion rate into rebaudioside A by the amount of the crude enzyme solution added of *Lactobacillus mali* 2 kinds, as shown in FIG. 2, when the crude enzyme solution was added by 5%, 15%, and 25%, respectively, the conversion rate of the crude enzyme solution of the *Lactobacillus mali* CJST01 strain was shown about 1.3 to 1.5 times higher than the crude enzyme solution of the *Lactobacillus mali* DSM20444 strain. Through this, compared to the *Lactobacillus mali* DSM20444 strain, when the *Lactobacillus mali* CJST01 strain was used, during preparing transglycosylated rebaudioside A of the same conversion rate, the Lactobacillus crude enzyme solution was required less, and therefore, it can be seen that it is advantageous in the transglycosylated rebaudioside A production process.

In addition, as a result of comparing chromatograms that the reaction solution after the addition reaction of 15% of the crude enzyme solution of the *Lactobacillus mali* 2 kinds was analyzed by HPLC, as shown in FIG. 3, it was confirmed that the peak of rebaudioside A, the raw material, was reduced and the peak of transglycosylated rebaudioside A increased after the reaction, when the crude enzyme solution of 2 kinds of the strains was added. Moreover, it was confirmed that the rebaudioside A peak of the reaction solution in which the *Lactobacillus mali* CJST01 strain crude enzyme solution was much more reduced compared to the reaction solution in which the *Lactobacillus mali* DSM20444 strain crude enzyme solution was added.

### Example 5. Evaluation of conversion rate of transglycosylated rebaudioside A by reaction condition

The conversion rate from rebaudioside A into transglycosylated rebaudioside A of the crude enzyme solution of the *Lactobacillus mali* CJST01 strain and the *Lactobacillus mali* DSM20444 strain depending on the reaction condition was evaluated.

Specifically, the rebaudioside A conversion rate was analyzed by HPLC by the same method as Example 4 under the conditions of addition of 5%, 10%, 15%, or 25% of the crude enzyme solution of the *Lactobacillus mali* CJST01 strain and *Lactobacillus mali* DSM20444 strain prepared in Example 3, substrate (rebaudioside A) concentration of 5%, 7%, or 10%, and reaction time of 6 hours, 24 hours or 48 hours, and it was shown in Table 3 and Table 4 below.

**[Table 3]**

| Rebaudioside A conversion rate by reaction conditions of *Lactobacillus mali* DSM20444 strain crude enzyme solution | | | | |
|---|---|---|---|---|
| Substrate concentration (Reb A) | Amount of crude enzyme solution added | Reaction time (h) | | |
| | | 6 | 24 | 48 |
| 5% | 5% | 15.5 | 47.9 | 69.2 |
| | 10% | 27.6 | 70.8 | 88.0 |
| | 15% | 39.3 | 84.1 | 93.2 |
| | 25% | 54.2 | 90.8 | 92.3 |
| 7% | 5% | 11.7 | 38.8 | 57.8 |
| | 10% | 21.6 | 59.5 | 80.2 |
| | 15% | 30.9 | 75.2 | 90.1 |
| | 25% | 44.6 | 87.7 | 93.4 |
| 10% | 5% | 8.3 | 29.0 | 42.4 |
| | 10% | 15.5 | 47.0 | 67.4 |
| | 15% | 21.7 | 59.5 | 79.5 |
| | 25% | 32.4 | 75.7 | 89.0 |

**[Table 4]**

| Rebaudioside A conversion rate by reaction conditions of *Lactobacillus mali* CJST01 strain crude enzyme solution | | | | |
|---|---|---|---|---|
| Substrate concentration (Reb A) | Amount of crude enzyme solution added | Reaction time (h) | | |
| | | 6 | 24 | 48 |
| 5% | 5% | 23.9 | 65.0 | 84.7 |
| | 10% | 40.1 | 84.5 | 93.3 |
| | 15% | 55.9 | 92.8 | 94.3 |
| | 25% | 72.0 | 93.0 | 91.2 |
| 7% | 5% | 17.7 | 52.0 | 73.3 |
| | 10% | 34.2 | 79.2 | 92.2 |
| | 15% | 44.8 | 87.5 | 93.4 |
| | 25% | 61.4 | 93.1 | 92.6 |
| 10% | 5% | 12.9 | 41.1 | 60.7 |
| | 10% | 23.0 | 62.1 | 81.6 |
| | 15% | 33.0 | 76.8 | 90.0 |
| | 25% | 49.2 | 89.1 | 91.5 |

As a result of confirming the rebaudioside A conversion rate of the crude enzyme solution of the *Lactobacillus mali* 2 kinds strains according to the reaction conditions, as shown in Table 3 and Table 4, in case of the substrate concentration of 10%, the amount of the crude enzyme solution added of 5%, 10% or 15%, and the reaction time of 6 to 48 hours, the *Lactobacillus mali* DSM20444 strain crude enzyme solution showed the transglycosylated rebaudioside A conversion rate of 8 to 80%, and the *Lactobacillus mali* CJST01 strain crude enzyme solution showed that of 13 to 90%. Based on these results, it can be confirmed that a higher conversion rate is shown at the same substrate concentration or in the same amount of the crude enzyme solution added, when the *Lactobacillus mali* CJST01 strain is used.

In addition, the reaction solution in which the *Lactobacillus mali* DSM20444 strain crude enzyme solution was added showed the rebaudioside A conversion rate of 90% under the conditions of the substrate concentration of 7%, the amount of the crude enzyme solution added of 15%, and the reaction time of 48 hours, and the reaction solution in which the *Lactobacillus mali* CJST01 strain crude enzyme solution was added showed the rebaudioside A conversion rate of 90% under the conditions of the substrate concentration of 10%, the amount of the crude enzyme solution added of 15%, and the reaction time of 48 hours. Through this, it can be seen that reaction solution with the same conversion rate can be obtained at a high substrate concentration, when the *Lactobacillus mali* CJST01 strain crude enzyme solution is used.

### Example 6. Analysis of transglycosylated rebaudioside A

In order to confirm how many glucoses were bound to transglycosylated rebaudioside A converted by adding the *Lactobacillus mali* strain crude enzyme solution, it was analyzed as follows.

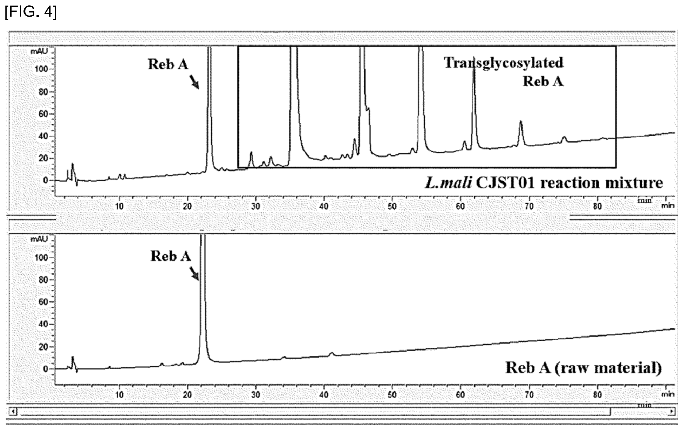

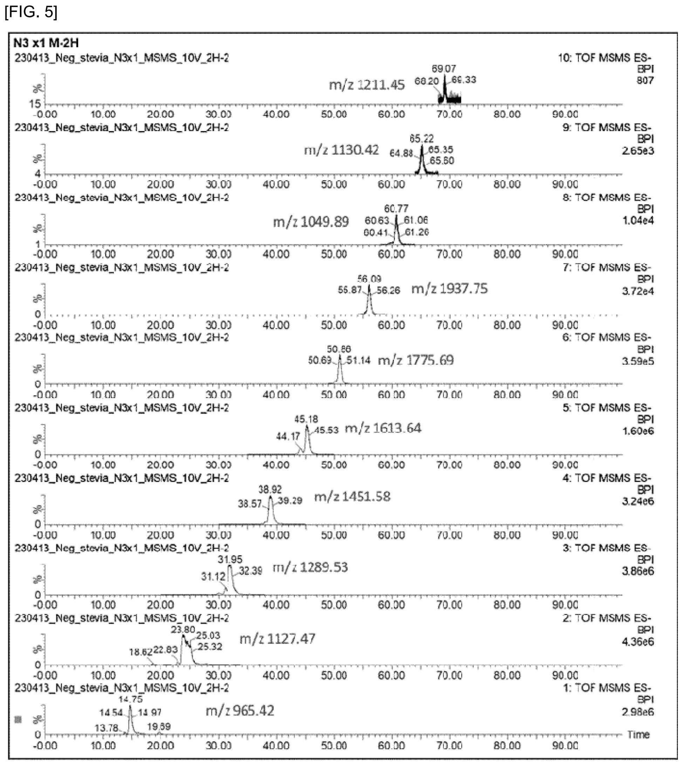

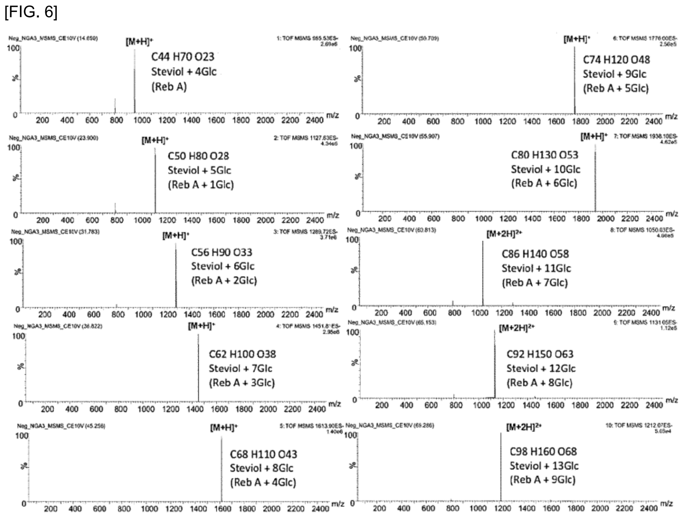

Specifically, rebaudioside A (Daepyung) and sucrose (CJ CheilJedang) were added to 50 µM sodium acetate buffer (pH 5.0) by 10% each, and the crude enzyme solution of the *Lactobacillus mali* CJST01 strain prepared in Example 3 was concentrated to 5 times, and was added at a concentration of 10%, and reacted at 40°C for 24 hours. After reacting, it was deactivated at 100°C, and the result of the HPLC analysis and the LC-MS analysis result under the conditions of Table 5 below were shown in FIGs. 4 to 6.

**[Table 5]**

| | | | |
|---|---|---|---|
| Detector | UV at 210 nm (4nm bw) | | |
| | Ref: 360 nm (100nm bw) | | |
| Column | NH2(4.6 * 250mm, 5um) | | |
| | ex. Agilent Zorbax NH2 | | |
| Column temp. | 40°C | | |
| Gradient | Time(min) | A (%) | B (%) |
| | 0 | 80 | 20 |
| | 2 | 80 | 20 |
| | 90 | 50 | 50 |
| | 91 | 80 | 20 |
| | 100 | 80 | 20 |
| Injection vol. | 12 uL | | |
| Flow rate | 1 mL/min | | |

As a result, as shown in FIGs. 4 to 6, it was confirmed that in the rebaudioside A of the reaction solution prepared using the *Lactobacillus mali* CJST01 strain crude enzyme solution, 1 to 9 glucoses were transferred to rebaudioside A.

### Example 7. Evaluation of transglycosylation activity by Lactobacillus mali strain crude enzyme solution by steviol glycoside

In order to evaluate the transglycosylation activity by the *Lactobacillus mali* strain crude enzyme solution by steviol glycoside, an experiment as follows was performed.

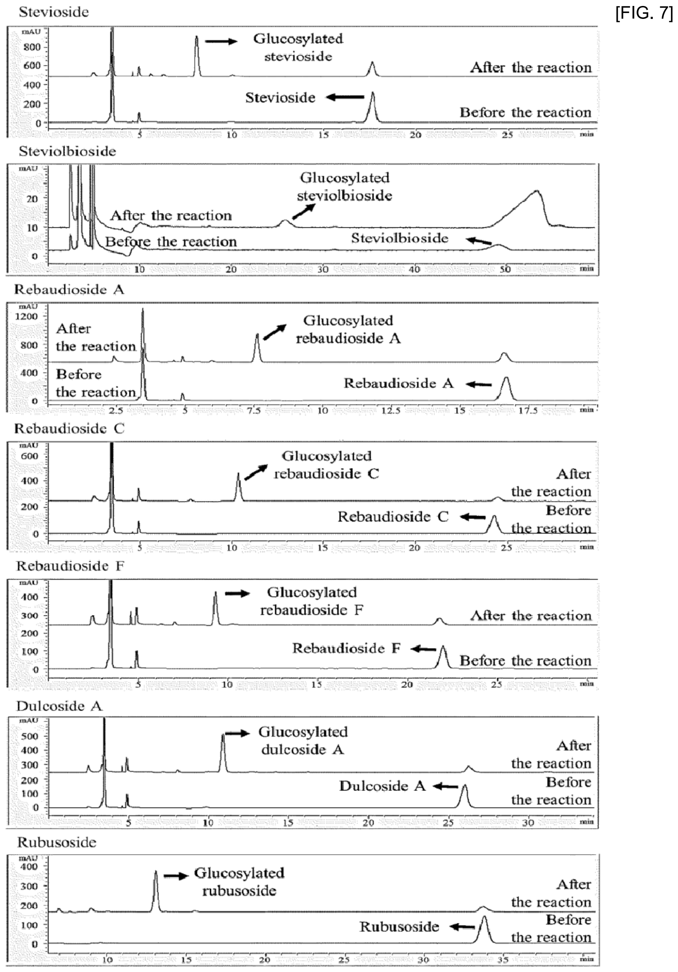

Specifically, 12 kinds of steviol glycosides (stevioside, rebaudioside A, B, C, D, E, F, M, N, dulcoside A, steviolbioside, rubusoside) and sucrose (CJ CheilJedang) were added to 50 µM sodium acetate buffer (pH 5.0) by 10% each, and the crude enzyme solution of the *Lactobacillus mali* 2 kinds of strains prepared in Example 3 was added and reacted at 40°C for 24 hours. After reacting, it was deactivated at 100°C, and the production of the transglycosylated steviol glycoside was confirmed by HPLC and it was shown in Table 6 and FIG. 7 below.

**[Table 6]**

| Steviol glycoside | DSM20444 | CJST01 |
|---|---|---|
| Rebaudioside A | ○ | ○ |
| Rebaudioside B | - | - |
| Rebaudioside C | ○ | ○ |
| Rebaudioside D | - | - |
| Rebaudioside E | ○ | - |
| Rebaudioside F | ○ | ○ |
| Rebaudioside M | - | - |
| Rebaudioside N | - | - |
| Stevioside | ○ | ○ |
| Dulcoside A | ○ | ○ |
| Steviolbioside | - | ○ |
| Rubusoside | O | ○ |

As a result, as shown in Table 6 and FIG. 7, it was shown that the *Lactobacillus mali* CJST01 strain crude enzyme solution had the transglycosylation activity for stevioside, rebaudioside A, C, and F, dulcoside A, steviolbioside, and rubusoside among 12 kinds of steviol glycosides. It was confirmed that Rebaudioside E showed the activity only in the *Lactobacillus mali* DSM20444 crude enzyme solution, whereas steviolbioside showed the activity only in the *Lactobacillus mali* CJST01 crude enzyme solution.

### Example 8. Sensory descriptive analysis of transglycosylated rebaudioside A aqueous solution

The CJST01 transglycosylated rebaudioside A (CJ) prepared by the method for preparing the transglycosylated steviol glycoside was analyzed for sensory differences between a comparative sample, rebaudioside M (RM), and alpha-1,4 transglycosylated rebaudioside A (A14) sample.

Specifically, the present evaluation was performed by descriptive analysis by recruiting professional sensory panels, and in advance, the concentration corresponding to the same sweetness level was calculated for 100 sweetness-sensitive female consumers between ages of 19 and 40. The sensory evaluation method used was Paired Comparison Test, and the final calculated concentration was shown in Table 7 below. Then, the consumers were asked to select the closest concentration by phasing the CJ and A14 sample concentrations into quintiles based on the RM concentration. The manufacturing method was to prepare the corresponding substance in a state that drinking was possible by dissolving it in drinking purified water at a set concentration (w/w), and the samples were provided into the consumer panels by 10 mL each in a transparent plastic cup, and two samples were randomly assigned to select the sample felt sweeter, and based on this, the sample in which statistical equivalence occurred among overall differences between the samples was calculated as the sample concentration for final descriptive analysis.

**[Table 7]**

| Sample list for evaluation for verification of sensory excellence of transglycosylated steviol glycoside | | |
|---|---|---|
| Classification | Calculated concentration | Note |
| CJ | 0.1197% | Alpha-1,6 transglycosylated rebaudioside A |
| RM | 0.0696% | Steviol glycoside |
| A14 | 0.2351% | Alpha-1,4 transglycosylated rebaudioside A |

The sensory evaluation through descriptive analysis was performed by using the sample concentration for descriptive analysis derived from the above as follows. The purpose of the sensory evaluation is to identify overall sensory profiles of each sweetener aqueous solution. The sensory evaluation was performed for 6 trained professional panels by repeating twice.

Specifically, samples were provided by 10 mL each in a transparent plastic cup at a room temperature, and the panels were provided with 3 cups per each sample and the sensory form was described. The evaluation was conducted for the randomly provided samples, and while investigating detailed flavor properties and the like, a rest period of about 2~3 minutes in the intervals of the samples was allowed to exclude the sensory influence between the samples. The evaluation items which the panels could feel were derived through preliminary sample evaluation, and detailed flavor/mouthfeel characteristics were provided as standard samples as shown below. The overall contents regarding this were shown in Table 8 below.

**[Table 8]**

| Attribute | | Definition | Reference |
|---|---|---|---|
| Flavor | Flower | Unique fragrant flower scent felt from a freesia flower | Sugar 8% aqueous solution + freesia flavor (Seoul Flavor) 0.00035% |
| | Honey | Unique flavor felt from honey | Miscellaneous honey flavor (Seoul Flavor) 0.02% aqueous solution |
| | Clove | Clover unique flavor | Clover flavor (Seoul Flavor) 0.005% aqueous solution |
| | Fishy smell of water | Fishy flavor from a fish tank or old water | - |
| | Metallicity | Iron flavor felt from a metallic substance such as iron, etc. | Liquid iron preparation, stainless steel spoon |
| Mouthfeel | cooling | Feeling that the mouth is cooled, after swallowing a sample | Mint flavor (Firmenich flavor) 0.002% aqueous solution |
| | Astringent sense | Feeling that tissues in the mouth is puckered or dried, felt when holding a sample in the mouth or swallowing it | Tannic Acid (Nam Yung Commercial Co., Ltd.)0.021 % aqueous solution |

As shown in Table 8, through sensory evaluation by descriptive analysis, as sensory characteristics of 5 kinds of the sweeteners, 'sweetness, bitterness, 5 flavor characteristics, 2 mouthfeel characteristics' were derived. For bitterness, standard substances (2 points: anhydrous caffeine 0.055%, 4 points: anhydrous caffeine 0.067%) were provided for each intensity.

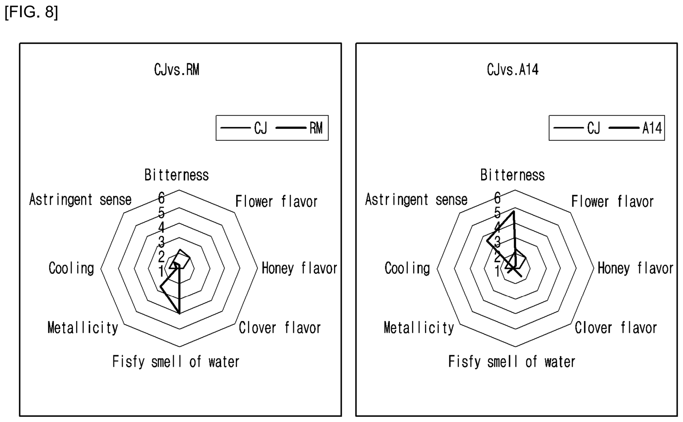

The intensity analysis result for the detailed flavor properties and the like was shown in Table 9 below, and the result of visualizing this by Spider Map was shown in FIG. 8.

**[Table 9]**

| Intensity¹⁾ analysis result for detailed properties | | | |
|---|---|---|---|
| **Detailed items** | **CJ** | **RM** | **A14** |
| Bitterness | 2.5 | 1.3 | **4.8** |
| Flower flavor | 1.5 | 1 | 1.3 |
| Honey flavor | 1 | 1 | 1 |
| Clover flavor | 1 | 1 | 1.8 |
| Fishy smell of water | 1 | **4** | 1 |
| Metallicity | 1.3 | **2.8** | 1.5 |
| Cooling | 1.5 | 1.3 | 1.3 |
| Astringent sense | 2 | 1.5 | **3.5** |

¹⁾ Means of two replicates. Data were scored on a 15 point category scale, where 1 - week intensity of the attribute and 15 = strong intensity of the attribute. Values within a row not sharing a superscript letter are significantly different (p<0.05 Duncan's multiple range test).

As shown in Table 8 and FIG. 8, RM showed chracteristics that the 'flavor of a fishy smell of water' was strongly expressed and the metallicity was strong, and the 'bitterness and astringent sense'showed characteristics of being weakly expressed compared to the A14 sample. In case of the A14 sample, characteristics that the 'bitterness/clove flavor/astringent sense' were relatively strong were shown. In case of the CJ sample, compared to the RM sample, the metallicity intensity was evaluated as weak, and the bitterness/astringent sense was evaluated at an equivalent level, and compared to the A14 sample, the bitterness/astringent sense properties were weakly expressed, and the intensity of other properties was investigated as equivalent.

In other words, through the above result, it could be confirmed that the sensory performance of the sweetener of the CJ sample had more excellent applicability than the comparative samples (less off-notes). Since sweeteners aim to replace sugar(sucrose), no the characteristics other than 'sweetness' are not required, and considering that this is applied to various foods, the mentioned characteristics are very important for universal use. Therefore, when combining the investigation results of the present descriptive analysis, it can be determined that the transglycosylated rebaudioside A by the present invention has been proven to have excellence as a food sweetener.

### Example 9. Evaluation of solubility of transglycosylated rebaudioside A

For evaluation of solubility of the steviol glycoside and the transglycosylated rebaudioside A, an aqueous solution in which 10 ml distilled water was added to 2g each of rebaudioside A (RebA), rebaudioside D (RebD), rebaudioside M (RebM), stevioside (STV) 2g and an aqueous solution in which 10 ml distilled water was added to 8g of transglycosylated rebaudioside A (RebA-Glu) were mixed and dissolved by a sonicator at 50°C for 60 minutes. They were incubated in a 10, 25, 40, 55 °C incubator for 4 days. Each aqueous solution was centrifuged at 12000 rpm for 10 minutes and the supernatant of 1 ml was dried in a 105 °C dry oven, and the solubility was measured and shown in Table 10. At this time, the transglycosylated rebaudioside A was obtained by preparing it as same as the manufacturing method of the transglycosylated rebaudioside A used in Example 7.

**[Table 10]**

| Temperature (°C) | Solubility (mg/ml) | | | | |
|---|---|---|---|---|---|
| | RebA | RebD | RebM | STV | RebA-Glu |
| 10 | 5 | 4 | 4 | 12 | 400↑ |
| 25 | 5 | 1 | 4 | 21 | 400↑ |
| 40 | 6 | 1 | 6 | 37 | 400↑ |
| 55 | 12 | 2 | 9 | 160↑ | 400↑ |

As a result, as shown in Table 10, it can be confirmed that the transglycosylated rebaudioside A had significantly increased solubility compared to rebaudioside A, a reaction substrate, and had exceptionally improved solubility than rebaudioside D, rebaudioside M, and stevioside, other steviol glycosides.

From the above description, those skilled in the art to which the present invention pertains will understand that the present invention can be implemented in other specific forms without changing its technical spirit or essential features. In this regard, the examples described above should be understood as illustrative and not restrictive in all aspects. The scope of the present invention should be interpreted as all changed or modified forms derived from the meaning and scope of the claims described below and equivalent concepts thereof are included in the scope of the present invention.

### [ACCESSION NUMBER]

Name of Depository Authority: Korea Research Institute of Bioscience and Biotechnology, Korean Collection for Type Cultures (KCTC)
Accession number: KCTC15433BP
Date of deposit: 20230508

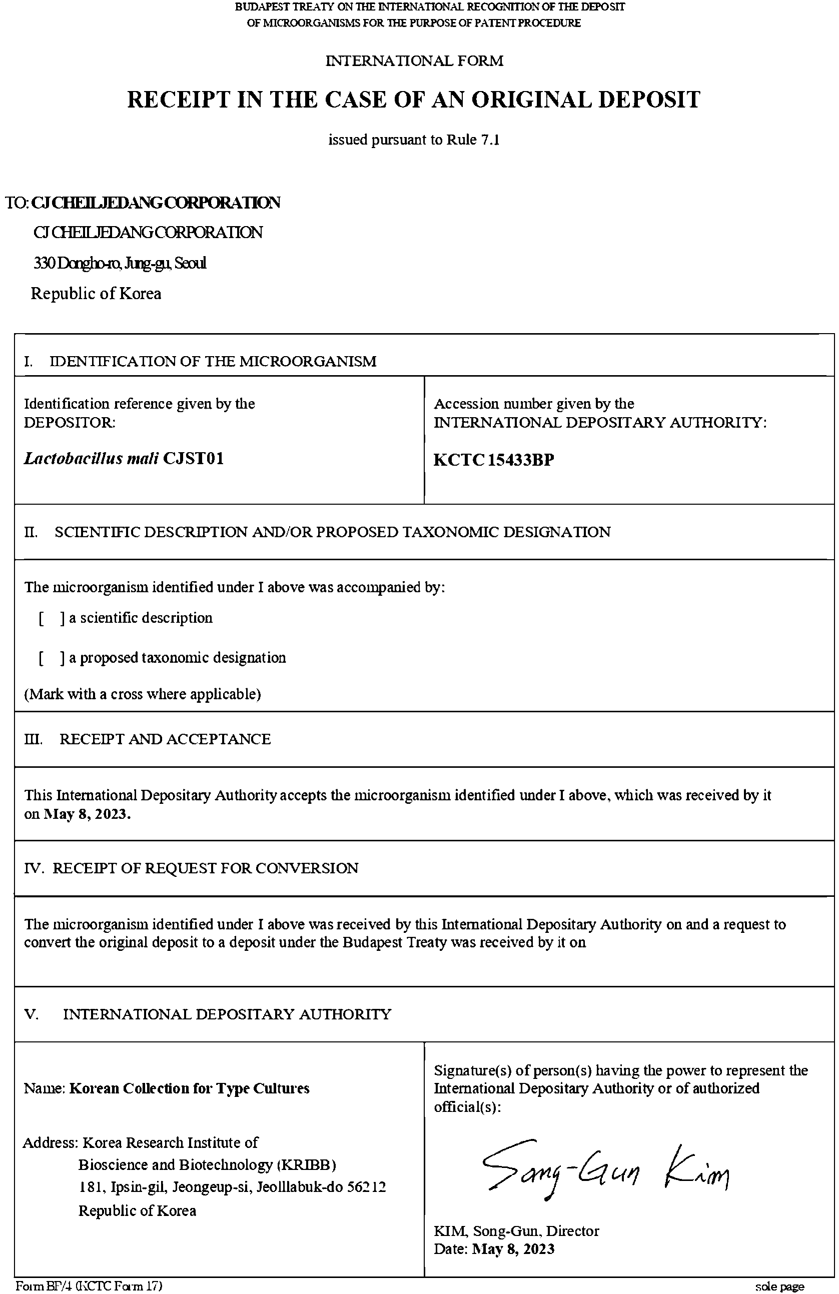
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CHEILJEDANG CORPORATION
CJ CHEILJEDANG CORPORATION
330 Dongho-ro, Jung-gu, Seoul
Republic of Korea

I. IDENTIFICATION OF THE MICROORGANISM
Identification reference given by the
DEPOSITOR:

Lactobacillus mali CJST01 | Accession number given by the
INTERNATIONAL DEPOSITARY AUTHORITY:

KCTC 15433BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on May 8, 2023.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: May 8, 2023

Form BP/4 (KCTC Form 17) sole page

## Claims

1. A *Lactobacillus mali* CJST01 strain deposited with accession number KCTC15433BP.

2. The strain according to claim 1, wherein the strain has the 16S rRNA sequence of SEQ ID NO: 1.

3. The strain according to claim 1, wherein the steviol glycoside is any one selected from the group consisting of stevioside, rebaudioside A, rebaudioside C, rebaudioside F, dulcoside A, steviolbioside, and rubusoside.

4. A method for preparing transglycosylated steviol glycosides, using the strain of claim 1.

5. The method for preparing transglycosylated steviol glycosides according to claim 4, comprising reacting sucrose and a steviol glycoside, in the presence of the *Lactobacillus mali* strain or a culture thereof.

6. The method for preparing transglycosylated steviol glycosides according to claim 4, wherein the steviol glycoside is any one selected from the group consisting of stevioside, rebaudioside A, rebaudioside C, rebaudioside F, dulcoside A, steviolbioside, and rubusoside.

7. The method for preparing transglycosylated steviol glycosides according to claim 6, wherein the steviol glycoside is a steviol glycoside added by linking a glucose by an α-(1, 6) bond through a glucose bound to the 19-OH position of the steviol glycoside.

8. The method for preparing transglycosylated steviol glycosides according to claim 7, wherein the steviol glycoside comprises 1 to 9 glucoses.

9. A transglycosylated steviol glycoside prepared by the method of any one claim of claim 4 to claim 8.

10. The transglycosylated steviol glycoside according to claim 9, wherein the steviol glycoside is any one selected from the group consisting of stevioside, rebaudioside A, rebaudioside C, rebaudioside F, dulcoside A, steviolbioside, and rubusoside.

11. A sweetener, comprising the transglycosylated steviol glycoside of claim 9.

12. A composition for producing transglycosylated steviol glycosides, comprising the strain of claim 1 or a culture thereof.
